# EUROPEAN PATENT APPLICATION

(11) **EP 4 817 659 A1**
(43) Date of publication of application: **07.10.2026**
(21) Application number: 26163499.2
(22) Date of filing: 10.03.2026
(51) Int. Cl.: A61M 16/00

(54) **SYSTEMS FOR VOLATILE ANESTHESIA DELIVERY**

(30) Priority: 03.04.2025 US 202519169956
(71) Applicant: GE Precision Healthcare LLC, Waukesha, WI 53188 (US)
(72) Inventor: KUZELKA, Russell J., Madison, 53718-6704 (US)
(74) Representative: Kilburn & Strode LLP

(57) **Abstract**

Various methods and systems are provided for anesthesia systems which may include a cartridge pump assembly including a cartridge containing a liquid anesthetic and a pump adapted to pressurize the liquid anesthetic through a pressure line and a reservoir. An injector may deliver the liquid anesthetic into a heater assembly. The system further includes a liquid flow valve configured to fluidly couple and decouple the cartridge and the injector.

## Description

### FIELD

Embodiments of the subject matter disclosed herein relate to volatile anesthesia delivery using anesthesia vaporizers.

### BACKGROUND

Anesthesia systems may vaporize volatile anesthetic liquids to deliver anesthetic vapors to a patient. Volatile anesthetics such as isoflurane and sevoflurane have pharmacological properties that allow well-tolerated and titrateable light-to-deep sedation. Use of volatile anesthetics may be helpful in both an operating room and intensive care unit (ICU) setting. For example, volatile anesthetics may be more quickly cleared from a patient's system than intravenously delivered sedation drugs, better allowing for periodic checks on sedated patients in the ICU.

### BRIEF DESCRIPTION

In one example, an anesthesia system includes a cartridge containing a liquid and a pump adapted to pressurize the liquid; an injector configured to deliver the liquid into a heater assembly; a liquid flow valve configured to fluidly couple and decouple the cartridge and the injector; and a pressure line and a reservoir positioned fluidly between the liquid flow valve and the injector. In this way, the liquid flow valve may be closed when the cartridge is being replaced with a new cartridge, preventing exposure of a user and the environment to liquid anesthesia. Further, the reservoir may store a volume of the liquid anesthesia such that when the liquid flow valve is closed, supply of the liquid anesthesia to the injector may not be interrupted for a period during which the current cartridge is removed and the new cartridge is positioned, allowing delivery of the anesthetic agent to a patient to continue during cartridge replacement without disruption. Further still, the cartridge pressurized anesthesia system may be compatible with various anesthetic agents having different properties such as boiling point and vapor pressure.

It should be understood that the brief description above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

### BRIEF DESCRIPTION OF THE DRAWINGS

The present disclosure will be better understood from reading the following description of non-limiting embodiments, with reference to the attached drawings, wherein below:
FIG. 1 shows a block diagram of a cartridge pressurized anesthesia system.
FIG. 2 shows an example of the cartridge pressurized anesthesia system including an internal cartridge.
FIG. 3 shows a diagram of the cartridge pressurized anesthesia system including the internal cartridge.
FIG. 4 shows a diagram of the cartridge pressurized anesthesia system including an external cartridge.
FIG. 5 shows an example of a heater assembly of the cartridge pressurized anesthesia system.
FIG. 6 shows a flowchart of a method for control of a pressure of the cartridge pressurized anesthesia system.
FIG. 7 shows a flowchart of a method for control of an injector of the cartridge pressurized anesthesia system.
FIG. 8 shows an example of the cartridge pressurized anesthesia system coupled to a ventilator.
FIG. 9 shows a detailed view of an interface of the ventilator and the cartridge pressurized anesthesia system.
FIG. 10 shows a flowchart of a method for operating the cartridge pressurized anesthesia system.

### DETAILED DESCRIPTION

The following description relates to systems and methods for a cartridge pressurized anesthesia system. Conventionally, anesthesia systems are coupled to a liquid sump holding the liquid anesthetic reagent. During operation, the liquid sump may demand manual refiling from bottles of the liquid anesthetic, thereby exposing technicians and the environment to uncontrolled doses of the anesthetic vapor. Further, coupling a volatile anesthesia system to a vaporizer in an intensive care unit (ICU) setting may demand use of a closed-cycle system wherein exhaled vapors are scrubbed of CO₂ and returned to the patient in addition to a scavenging system that is coupled to the suction available in an ICU. Further still, conventional anesthesia systems may not enable providing therapy and refilling the liquid sump to occur simultaneously, leading to disruptions in delivery of anesthesia to the patient.

The inventors hereby recognize the disadvantages discussed above and hereby attempt to address these disadvantages with the cartridge pressurized anesthesia system. By introducing the liquid anesthetic reagent in a cartridge, such as a syringe, pouring of liquid anesthesia reagents by technicians may be avoided. Instead, cartridges may be prefilled in a controlled environment before they are handled by technicians in an ICU or operating room setting. Further, the cartridge pressurized anesthesia system may be configured for circular anesthesia systems and may include an integrated CO₂ removal system that is adapted for patients with various tidal volumes (e.g., including low lung flow) and/or use of a humidifier. Additionally, the cartridge may be replaced with a full cartridge to replenish the supply of anesthesia without disrupting delivery of anesthesia to the patient.

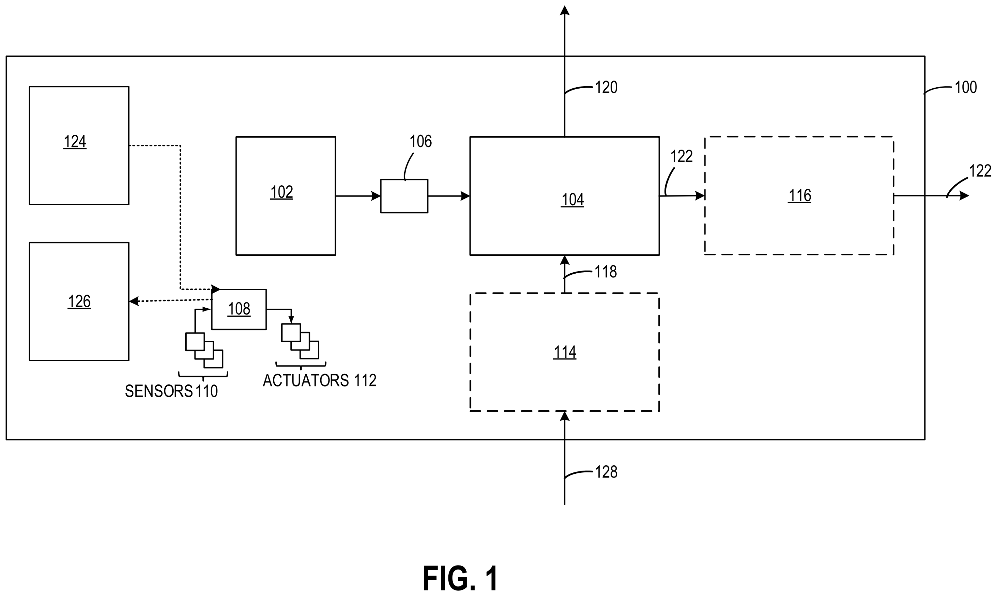

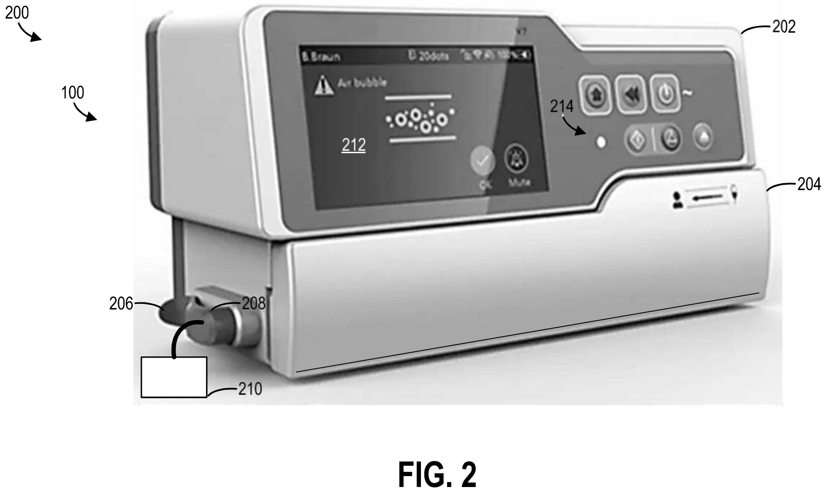

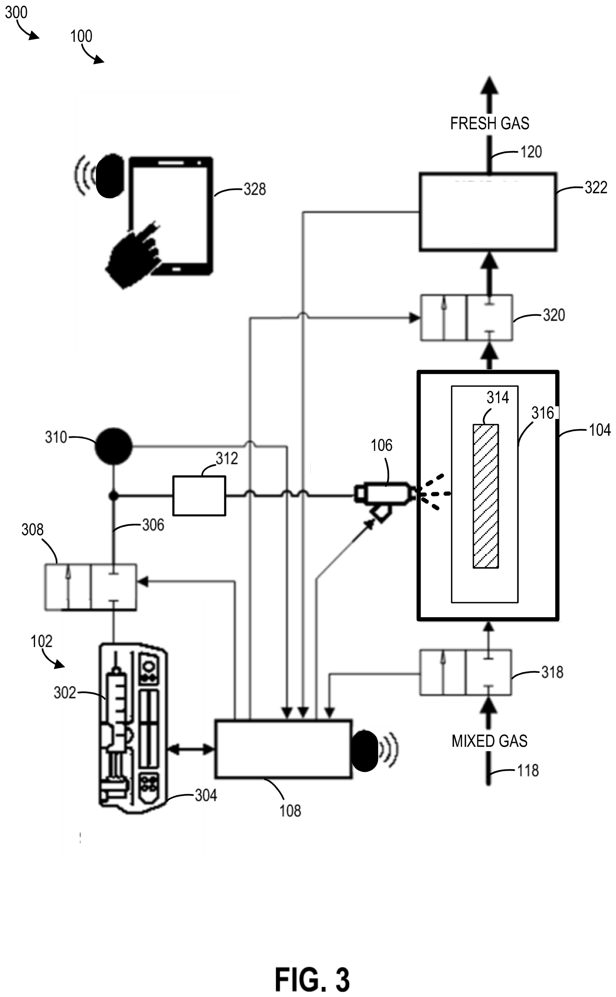

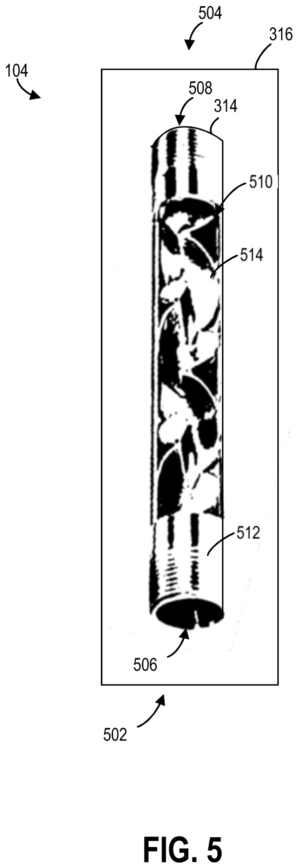

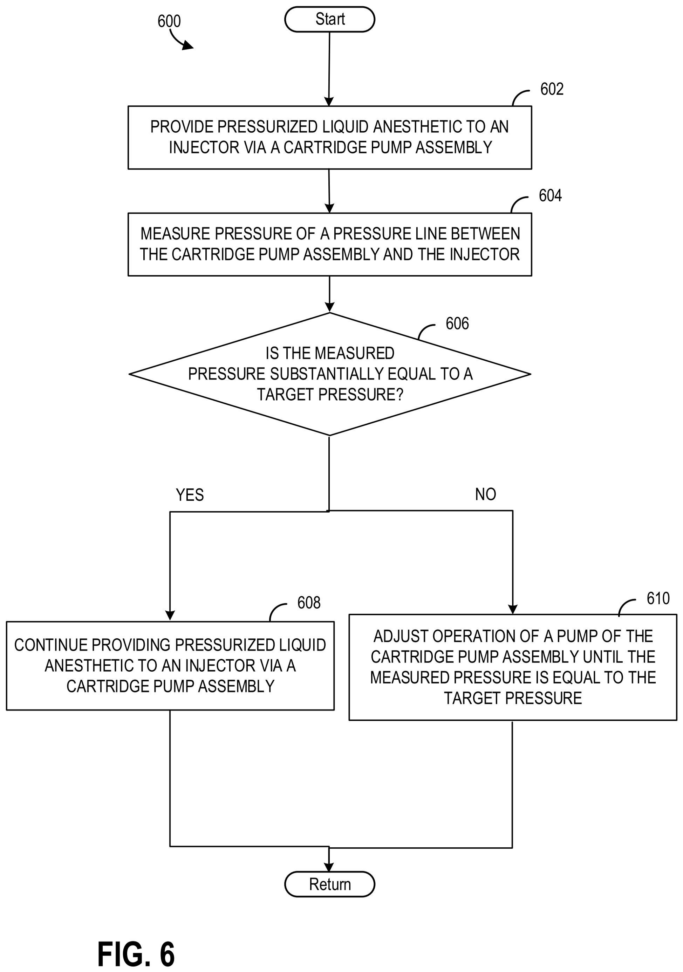

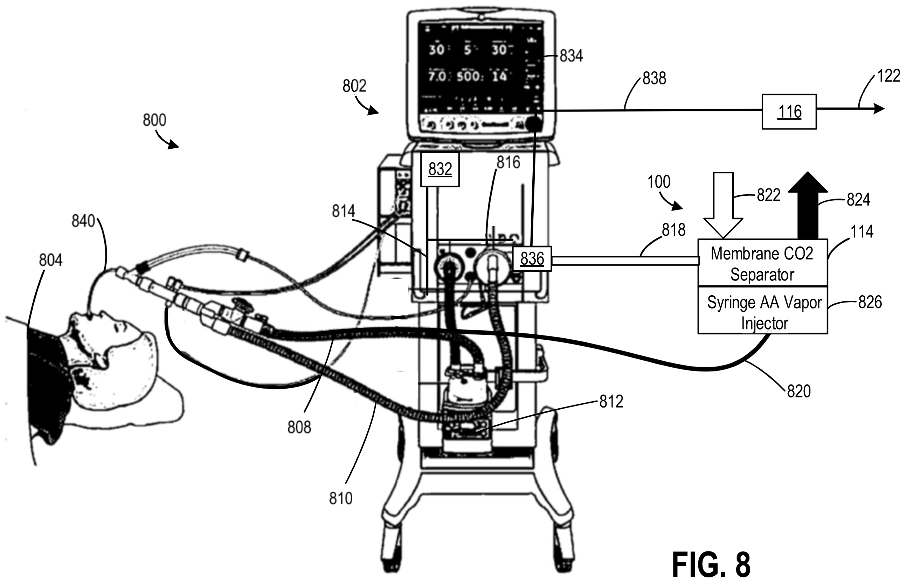

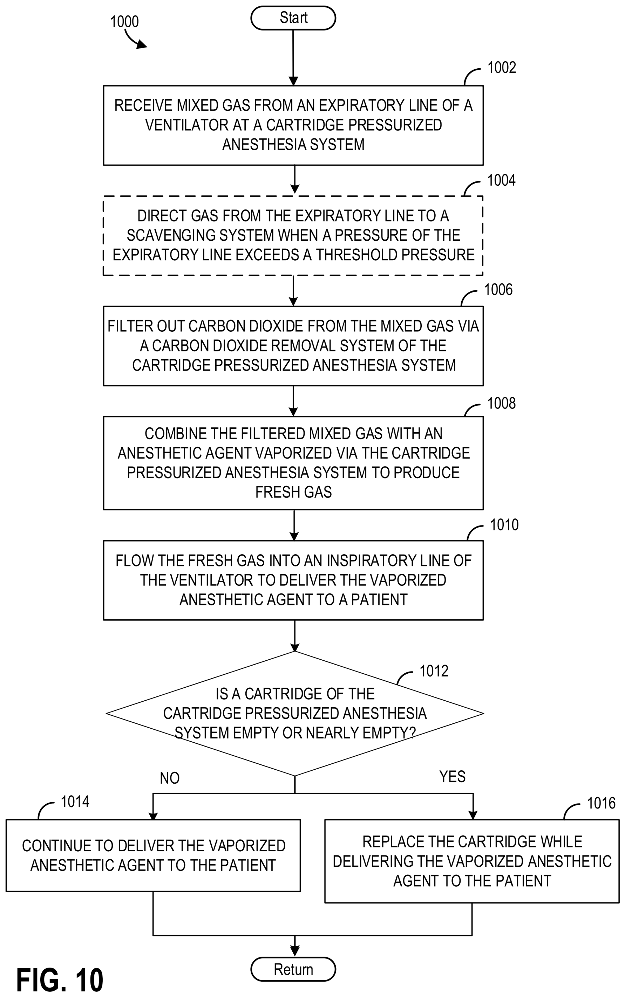

The cartridge pressurized anesthesia system may be configured to receive mixed gas, vaporize liquid anesthesia, and output fresh gas as shown in FIG. 1. The cartridge pressurized anesthesia system may be further configured to remove CO₂ from the mixed gas. In one example, the cartridge pressurized anesthesia system may be a self-contained system including a display, and inlets/outlets configured to couple to the source and destination of the mixed gas and fresh gas respectively as shown in FIG. 2. Diagrams showing flow of anesthetic liquid and gas in addition to sensors and actuators of the cartridge pressurized anesthesia are shown in FIGS. 3-4. An example of a heating system of the cartridge pressurized anesthesia system is shown in FIG. 5. Dosing of the anesthetic agent may be accurately controlled by actuators of the cartridge pressurized anesthesia system. Flowcharts of methods for controlling the actuators based on feedback from the sensors of the cartridge pressurized anesthesia system are shown in FIGS. 6-7. As one example, gas may be circulated through the cartridge pressurized anesthesia system by a ventilator as diagramed in FIGS. 8-9. A flowchart of a method for operating the cartridge pressurized anesthesia system is shown in FIG. 10.

Turning now to FIG. 1, a block diagram of the cartridge pressurized anesthesia system 100 is shown. Cartridge pressured anesthesia system 100 may include a cartridge pump assembly 102. The cartridge pump assembly 102 may include a cartridge and a pump configured to deliver a volatile liquid anesthesia reagent to a vaporizer at a set desired pressure. The cartridge may include an internal volume configured to hold the volatile liquid anesthesia reagent and prevent vapors from escaping. Further, the cartridge may mechanically couple to the pump configured to adjust the internal volume of the cartridge to pressurize the volatile liquid anesthesia reagent. As one example described further below, the cartridge may be a syringe and the pump may be a linear syringe pump.

Cartridge pump assembly 102 may be fluidly coupled to a heater assembly 104 via an injector 106. Injector 106 may receive pressurized volatile liquid anesthesia reagent from the cartridge pump assembly 102 and disperse droplets of the pressurized volatile liquid anesthesia reagent into heater assembly 104. Heater assembly 104 may be configured to vaporize the liquid reagent which originated in the cartridge pump assembly 102 into a gas and mix the incoming mixed gas 118 therewith. The mixed gas 118 (e.g., O₂/N₂ mixture) may combine with the vaporized anesthesia within heater assembly 104 and heater assembly 104 may output fresh gas 120 (e.g., a mixture of mixed gas and anesthesia reagent).

Cartridge pressurized anesthesia system 100 may further include a controller 108 communicatively coupled to sensors 110 and actuators 112 of the cartridge pressurized anesthesia system 100. Sensors 110 may include one or more of a pressure sensor, a flow rate sensor, a temperature sensor, and a sensor configured to detect a concentration of anesthetic agent in the fresh gas 120 (e.g., a nondispersive infrared (NDIR) sensor). Actuators 112 may include the injector 106 and the pump of the cartridge pump assembly 102. Controller 108 may be a microcomputer, including a microprocessor, input/output ports, and non-volatile electronic storage medium for executable programs and calibration values. Controller 108 may be programmed with computer readable data representing instructions executable to perform the methods described further below as well as others.

Cartridge pressurized anesthesia system 100 may further include a user interface 124 communicatively coupled to controller 108. User interface 124 may be configured for a user to input operating parameters to the controller 108. In one example, user interface 124 may be integrated into a housing of cartridge pressurized anesthesia system 100. Additionally or alternatively, user interface 124 may include an input device, such as keyboard, touch screen, or the like, outside of the housing and communicatively coupled to controller 108. In some examples, user interface 124 may be incorporated into a portable device such as tablet, laptop, smartphone, or the like, including instructions to communicate with the controller 108 via wired or wireless (e.g., WiFi or Bluetooth^{®}) communication.

Cartridge pressurized anesthesia system 100 may include a display 126 communicatively coupled to controller 108. Display 126 may be configured to display operating status of the cartridge pressurized anesthesia system 100 to the user. In some examples, display 126 may be integrated with the housing of cartridge pressurized anesthesia system 100. Additionally or alternatively, display 126 may be outside of the housing and communicatively coupled to controller 108. For example, a tablet, laptop, external monitor, or the like may be configured as display 126 and may be communicatively coupled to controller 108 via wired or wireless communication. In some examples, a single external computing device, such as a tablet, laptop, smartphone, or the like, may include the display 126 and the user interface 124.

Cartridge pressurized anesthesia system 100 may optionally include a CO₂ removal system 114. CO₂ removal system 114 may be positioned upstream of the heater assembly 104 with respect to the flow of gas. CO₂ removal system 114 may be configured to receive mixed gas 128 exhaled by a patient which may include CO₂ in addition to anesthetic agent and output the mixed gas 118 with reduced CO₂ (e.g., without CO₂). CO₂ removal system 114 may include a CO₂ filtration system, such as a membrane filtration system, as described further with reference to FIG. 8 below.

Cartridge pressurized anesthesia system 100 may further optionally include a scavenging system 116. Scavenging system 116 may be configured to receive excess mixed gas and waste anesthetic gas 122 not delivered to the patient. Scavenging system 116 may include reagents to sequester the waste anesthetic gas 122 for removal and appropriate disposal.

Turning now to FIG. 2, a non-limiting example of a housing 202 of cartridge pressurized anesthesia system 100 is illustrated in a perspective view 200. Housing 202 may enclose components of cartridge pressurized anesthesia system 100, including injector 106, heater assembly 104, and controller 108. In at least some examples, housing 202 may further enclose cartridge pump assembly 102. Housing 202 may include a door 204. Door 204 may be configured to open, making the cartridge of cartridge pump assembly 102 selectively accessible to the user in an example where cartridge pump assembly 102 is housed by housing 202. Other components of the cartridge pump assembly 102, injector 106, and heater assembly 104 may not be accessible to the user and may be controlled automatically by controller 108. In this way, cartridge pressurized anesthesia system 100 may be easy to use by a technician with a minimal amount of training.

Housing 202 may include a gas inlet 206 and a gas outlet 208. Gas inlet 206 may be configured to receive mixed gas. For example, gas inlet 206 may couple to a ventilator and may receive mixed gas output by a ventilator and/or circulated mixed gas exhaled by a patient (e.g., mixed gas 128 of FIG. 1). In examples where the mixed gas is exhaled gas (e.g., gas exhaled by the patient), the CO₂ removal system (e.g., CO₂ removal system 114 of FIG. 1) may be fluidly coupled to gas inlet 206. Gas outlet 208 may be configured to output fresh gas. For example, gas outlet 208 may be fluidly coupled to the ventilator and may output fresh gas into the inspiratory line of a ventilator.

Cartridge pressurized anesthesia system 100 may further include an adjustable pressure limiting valve 210 fluidly coupled to gas outlet 208. Adjustable pressure limiting valve 210 may be fluidly coupled to an expiratory line of the ventilator upstream of gas inlet 206. As such, adjustable pressure limiting valve 210 may limit pressure in the expiratory line, preventing lungs of the patient from being subject to pressures over a threshold pressure above which the lungs may be strained. For example, the threshold pressure may be adjusted according to characteristics of the patient. Adjustable pressure limiting valve 210 may relieve pressure automatically, responsive to experiencing pressure at or above the threshold, preventing overpressurization of the expiratory line. Thus, the adjustable pressure limiting valve 210 may be a pressure relief valve. For example, adjustable pressure limiting valve 210 may direct gas out of the expiratory line when pressure thereof exceeds the threshold. In such an example, the gas directed out of the expiratory line may be directed to a scavenging system as described further below. In one example, using FIG. 1 as a reference, in response to a pressure sensor detecting the threshold pressure is exceeded, the controller 108 of FIG. 1 may send a command signal to adjust pressure limiting valve 210 such that gas is directed out of the expiratory line. Alternatively, pressure limiting valve 210 may be self-actuating. For example, pressure limiting valve 210 may comprise a mechanical mechanism (e.g., including a spring or the like) which when overcome by pressure over the threshold pressure causes pressure limiting valve 210 to adjust such that gas is directed out of the expiratory line. In this way, pressure limiting valve 210 may automatically reduce pressure in the expiratory line to at or below the threshold pressure. Pressure limiting valve 210 is shown schematically as being positioned outside of housing 202 in FIG. 2, though in other examples, pressure limiting valve 210 may be enclosed in housing 202.

Housing 202 may include an integrated display 212. Integrated display 212 may be an example of display 126 as discussed above with respect to FIG. 1. Integrated display 212 may display a graphic user interface (GUI) output by the controller 108. In some examples, integrated display 212 may be a touch screen display and may also be an example of user interface 124. Housing 202 may additionally include physical interface 214. Physical interface 214 may include buttons depressible by the user to actuate actuators of cartridge pressurized anesthesia system 100. Further, physical interface 214 may be configured for user interaction with GUI displayed on integrated display 212.

Turning now to FIG. 3, a diagram 300 of an example of cartridge pressurized anesthesia system 100 is shown, including cartridge pump assembly 102, injector 106, and heater assembly 104.

Cartridge pump assembly 102 may include a cartridge 302 mechanically coupled to a pump 304. In one example, as shown in FIG. 3, cartridge 302 may be a syringe and pump 304 may be a syringe pump, although other variable volume cartridges and corresponding pumps are also considered as discussed above with respect to FIG. 1. Cartridge 302 may be self-sealing. For example, cartridge 302 may be configured to automatically seal from the environment in response to being decoupled from pump 304. Cartridge 302 may be configured to be stable (e.g., not degraded) under the operating and storage pressures and temperatures. For example, the material forming the cartridge may be chemically compatible with and not permeable to volatile liquid anesthetic agents in the demanded pressure and storage temperature ranges. Pump 304 may be communicatively coupled to controller 108. Cartridge 302 may be reversibly mechanically coupled to pump 304. Herein, reversibly mechanically coupled components may be coupled and decoupled without degradation of the components and without specialized tools or equipment.

Cartridge 302 may be fluidly coupled to a pressure line 306. A liquid flow valve 308 (e.g., a pneumatic valve) may be positioned between cartridge 302 and pressure line 306 with respect to the flow of liquid anesthetic agent. Cartridge 302 may be selectively fluidly coupled to pressure line 306 via liquid flow valve 308. Liquid flow valve 308 may be communicatively coupled to controller 108. Controller 108 may include instructions to close liquid flow valve 308 to fluidly decouple cartridge 302 from pressure line 306 in response to cartridge 302 being empty or nearly empty of liquid anesthetic agent. Closing liquid flow valve 308 may maintain liquid pressure within pressure line 306 and prevent leakage of liquid anesthetic agent to the environment while the current cartridge (e.g., empty or nearly empty cartridge) is exchanged for a new cartridge (e.g., full cartridge).

Pressure line 306 may fluidly couple cartridge 302 to injector 106. A pressure sensor 310 may be configured to a sense a liquid pressure within pressure line 306. Pressure sensor 310 may be communicatively coupled to controller 108. The pressure within pressure line 306 may be affected by liquid from cartridge 302 being forced into pressure line 306 by pump 304 changing the volume of cartridge 302. As one example, a pressure within pressure line 306 may be maintained in a range of between 20 psi and 30 psi. The pressure within pressure line 306 may thus be adjusted by adjusting (e.g., operating) pump 304. In this way, cartridge pressurized anesthesia system 100, specifically cartridge pump assembly 102, may be adaptable for use with a plurality of different volatile liquid anesthesia agents, each having a different boiling point/vapor pressure and demanding different pressures within pressure line 306. In one example, the cartridge 302 may include an identifier specific to the volatile liquid anesthesia agent included therein, and pump 304 may be configured to read the identifier and communicate the identity of the volatile liquid anesthesia agent to controller 108 to automatically adjust a target pressure of pressure line 306 accordingly. For example, the identifier may be a radiofrequency identification (RFID) tag. Controller 108 may actuate pump 304 according to sensor feedback in order to maintain the target pressure within pressure line 306 as described further below with reference to FIG. 6.

A reservoir 312 may be fluidly coupled to pressure line 306. Reservoir 312 may be fluidly positioned between liquid flow valve 308 and injector 106. Reservoir 312 may store a non-zero volume of pressurized liquid anesthetic reagent. Thus, while liquid flow valve 308 is closed and the cartridge 302 is changed for a full cartridge, reservoir 312 may supply injector 106 with pressurized liquid anesthetic. By doing so, anesthetic agent is actively delivered to a patient concurrently with the exchange of the cartridge 302. In this way, supply of liquid anesthetic agent may be replenished without interrupting sedation of the patient as may occur when using other systems that do not include a replaceable cartridge that is selectively fluidly coupled to a reservoir. Reservoir 312 may be positioned anywhere along pressure line 306 between liquid flow valve 308 and injector 106.

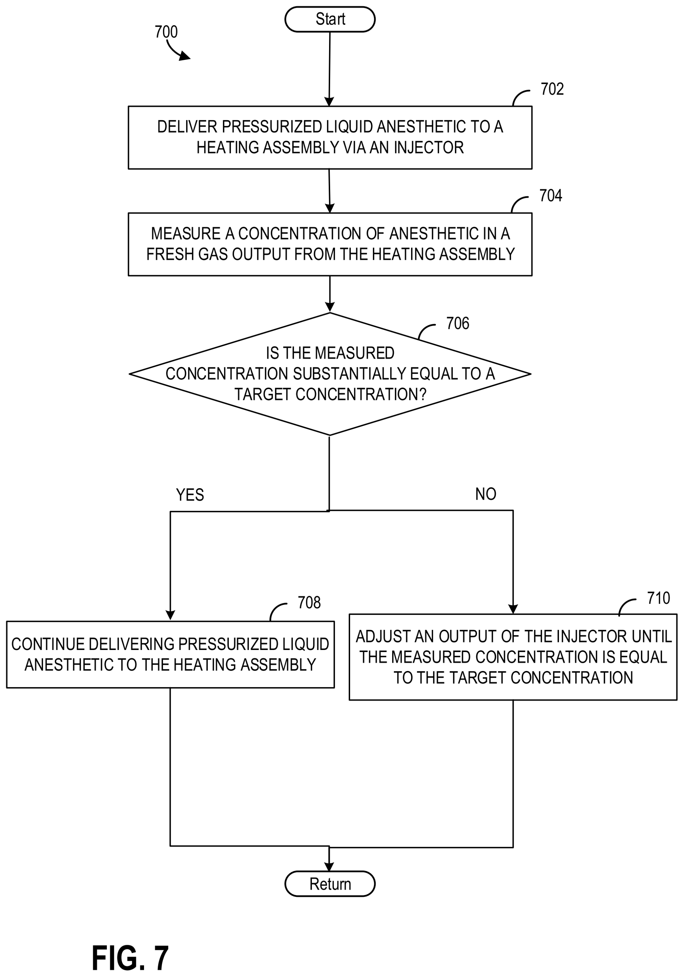

Injector 106 may be positioned fluidly between pressure line 306 and heater assembly 104. Injector 106 may be configured to receive pressurized liquid anesthetic from pressure line 306 and spray droplets of liquid anesthetic into the mixed gas flowing through heater assembly 104. As one example, injector 106 may be the same or similar to an automotive fuel injector. Injector 106 may be communicatively coupled to controller 108. Controller 108 may actuate injector 106 at a rate (e.g., on a duty cycle) and for a duration to control an amount (e.g., concentration) of anesthetic reagent in fresh gas 120 output by cartridge pressurized anesthesia system 100. Controller 108 may control a duty cycle of the injector 106. Controller 108 may actuate injector 106 based on the identifier of the cartridge of cartridge pump assembly 102. Controller 108 may further actuate injector 106 based on sensor feedback, as described further below with reference to FIG. 7. Thus, cartridge pressurized anesthesia system 100 may be adaptable for use with a plurality of different volatile liquid anesthesia agents, each demanding a different concentration in fresh gas 120.

Heater assembly 104 may include a heating element 316 thermally coupled to a heating passage 314. Heating element 316 may be communicatively coupled to controller 108. Heating element 316 may provide the thermal energy to volatilize (e.g., vaporize) the liquid anesthetic agent. In one example, heating element 316 may be a self-regulating heater. In some examples, heating element 316 may be a ceramic positive-temperature-coefficient (PTC) heating element. Heater assembly 104 may be controlled by controller 108. For example, controller 108 may adjust an amount of thermal energy output by heating element 316. In this way, cartridge pressurized anesthesia system 100 may be adaptable for use with a plurality of different volatile liquid anesthesia agents, each having a different boiling point and thus demanding a different temperature within heater assembly 104.

Heating passage 314 may be hollow chamber with at least a first end configured to receive mixed gas 118 and a second end configured to output fresh gas 120. Heating passage 314 may be configured to transfer thermal energy from heating element 316 to the droplets of liquid anesthetic agent output by injector 106. Heating passage 314 may be formed of a thermally conductive material. Heating passage 314 may include a gas inlet configured to receive mixed gas 118 and a gas outlet configured to output fresh gas 120. In some examples, injector 106 may disperse liquid anesthetic agent droplets into the gas inlet, tangential with the flow of mixed gas 118. In alternate examples, heating passage 314 may include an opening configured to receive the dispersed liquid anesthetic agent droplets at a non-zero angle with respect to the flow of mixed gas 118 through heating passage 314. An example of heater assembly 104 is described further below with respect to FIG. 5.

Cartridge pressurized anesthesia system 100 may include an input gas valve 318 and an output gas valve 320. Input and output gas valves 318 and 320 may be pneumatic valves, in one example. Input gas valve 318 may be positioned between a mixed gas inlet (e.g., gas inlet 206 of FIG. 2) and the gas inlet of heater assembly 104 with respect to the flow of gas. Output gas valve 320 be positioned between the gas outlet of heater assembly 104 and the fresh gas outlet (e.g., gas outlet 208 of FIG. 2) with respect to the flow of gas. In this way, input gas valve 318 and output gas valve 320 may be positioned respectively upstream and downstream of heater assembly 104. Input gas valve 318 and output gas valve 320 may each be communicatively coupled to controller 108. In one example, controller 108 may open input gas valve 318 and output gas valve 320 when a gas source and gas destination, such as a ventilator, are fluidly coupled to the cartridge pressurized anesthesia system 100. As another example, when cartridge pressurized anesthesia system 100 is not fluidly coupled to a gas source and gas destination, controller 108 may close input gas valve 318 and output gas valve 320 to prevent gaseous anesthetic agent from exiting the heater assembly 104. Input gas valve 318 may be positioned downstream of adjustable pressure limiting valve 210 of FIG. 2.

Cartridge pressurized anesthesia system 100 may include an anesthetic agent sensor 322. Anesthetic agent sensor 322 may be configured to determine an amount (e.g., concentration) of anesthetic agent in fresh gas 120 output form cartridge pressurized anesthesia system 100 and may be communicatively coupled to controller 108. In one example, anesthetic agent sensor 322 may be a NDIR detector configured for the region of the infrared spectrum absorbed by the anesthetic agent. Anesthetic agent sensor 322 may be configured to determine a concentration of various anesthetic agents, each absorbing a different wavelength. Anesthetic agent sensor 322 sensor may be calibrated to determine a concentration of anesthetic agent in fresh gas 120. Anesthetic agent sensor 322 may be used as part of feedback loop to control injector 106 and consequently the amount of anesthetic agent delivered to the patient. Specifically, controller 108 may actuate injector 106 based on signals received from anesthetic agent sensor 322 as described further below regarding FIG. 7.

Cartridge pressurized anesthesia system 100 may include a user interface and display 328. In one example, the user interface and display 328 may be an integrated device configured to display and receive user input. For example, user interface and display 328 may be one or more of a personal computer, laptop, tablet, smartphone, or the like. In one example, user interface and display may be integrated into a housing of the cartridge pressurized anesthesia system 100 as shown in FIG. 2. User interface and display 328 may be communicatively coupled to controller 108. In some examples, user interface and display 328 may be wirelessly communicatively coupled to controller 108. Alternatively, the user interface and display 328 may be communicatively coupled to controller 108 via one or more electrically conductive wires.

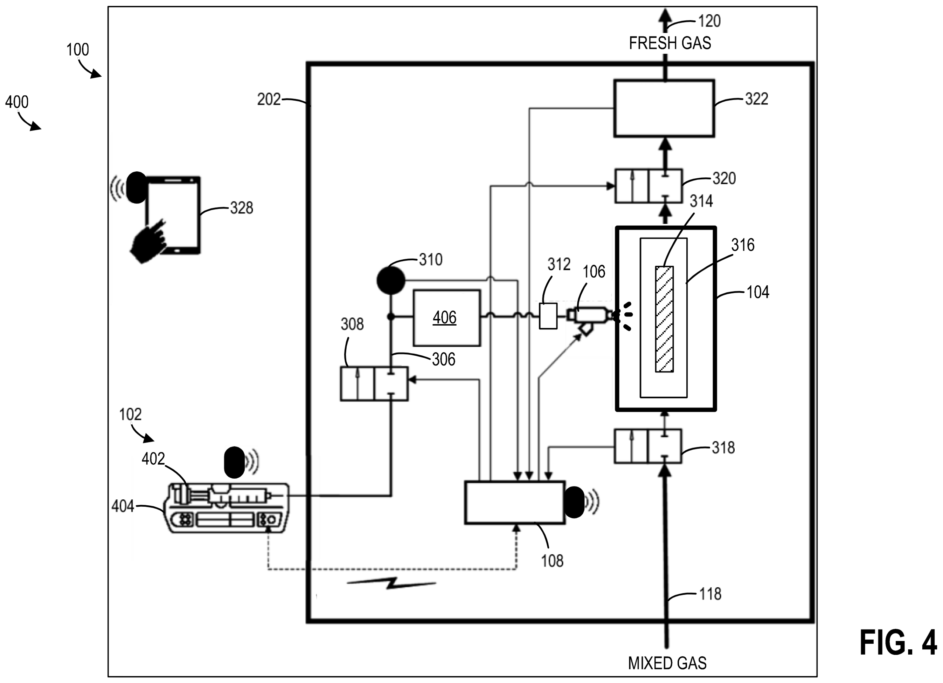

Cartridge pump assembly 102 may be positioned internally, such as within a housing (e.g., housing 202) further enclosing heater assembly 104, controller 108, injector 106, and reservoir 312. Therefore, pump 304 may be configured integrally within the cartridge pressurized anesthesia system 100 such that pump 304 is not removable from the housing. Pump 304 may not be directly accessible by a user, other than an opening to insert and remove cartridge 302. Alternatively, as described further with regard to FIG. 4, cartridge pump assembly 102 may be positioned externally, outside of the housing. Cartridge pump assembly 102 may further be removably coupled to pressure line 306 in such an example, allowing compatibility of cartridge pressurized anesthesia system 100 with a conventional pump.

Turning to FIG. 4, a diagram 400 of another example of cartridge pressurized anesthesia system 100 is shown, including cartridge pump assembly 102, injector 106, and heater assembly 104. The diagram 400 includes various components described above with regard to the diagram 300 in FIG. 3 which may be referenced and may not be reintroduced.

Cartridge pump assembly 102 may include a cartridge 402 and a pump 404. Cartridge 402 may have similar characteristics to as described above with regard to cartridge 302 in FIG. 3, including self-sealing ability, variable volume, and stability (e.g., not subject to degradation) under the operating and storage pressures and temperatures. Likewise, pump 404 may have similar characteristics to as described above with regard to pump 304 in FIG. 3, including ability to detect an identifier of cartridge 402 and change the volume of cartridge 402. However, cartridge 402 and pump 404 may be externally positioned (e.g., outside of housing 202). Housing 202 may include a port configured to removably fluidly couple pressure line 306 with cartridge 402. Pump 404 may be communicatively coupled to controller 108 via wired and/or wireless connection. Cartridge 402 and pump 404 being external may allow user provided pumps to be used to deliver pressurized flow of liquid contained by cartridge 402 to the injector 106 via control loops controlled by controller 108. Rather than being integral with components housed within housing 202, in the example of FIG. 4, cartridge pump assembly 102 may be removably physically coupled to pressure line 306 and selectively communicatively coupled to controller 108. For example, a communicative coupling between pump 404 and controller 108 may be formed on demand (e.g., by forming a physical wired connection or establishing a wireless connection). However, pump 404 may be removed from housing 202 and communication between pump 404 and controller 108 may be stopped such that pump 404 may be used in other systems when cartridge pressurized anesthesia system 100 is not in use.

In this way, cartridge pressurized anesthesia system 100 may be compatible with various configurations of cartridge pump assembly 102 when externally located, allowing selection according to preference of the user. For example, a user may desire a larger capacity cartridge so that replacement thereof is demanded less frequently. Thus, greater flexibility in volume of the cartridge may be allowed in external configurations such as shown in FIG. 4 compared to internal configurations such as shown in FIG. 3. However, an internal configuration where the pump is integral (e.g., not removable) may be easier for a user to operate (e.g., by eliminating demand for manual pump coupling). Therefore, uses for both configurations (e.g., internal and external pump) exist.

Cartridge pressurized anesthesia system 100 may further include a liquid flow sensor 406. Liquid flow sensor 406 may be configured to measure a flow rate of anesthetic liquid through the pressure line 306. Liquid flow sensor 406 may be positioned upstream or downstream of reservoir 312. Liquid flow sensor 406 may be positioned upstream or downstream of pressure sensor 310. In some examples, liquid flow sensor 406 is omitted, as the flow rate through pressure line 306 may be deduced (e.g., approximated) from other variables communicated to controller 108 such as the pressure monitored by pressure sensor 310, tubing diameter and/or operation of pump 404 (e.g., rate of change of cartridge volume). In some examples, liquid flow sensor 406 may be included in the internal configuration shown in FIG. 3.

For example, the flow rate of anesthetic liquid through pressure line 306 may be calculated based on Pouiseuille's Law for laminar flow (equation 1). $Q = \frac{{πr}^{4} Δ P}{8 μL}$ (1) In equation 1, Q is the volumetric flow rate, π is the mathematical constant (e.g., ≈ 3.14159...), *r* is the pipe radius (e.g., tubing radius), Δ*P* is the pressure difference, µ is the dynamic viscosity of the fluid (e.g., anesthetic liquid), and L is the length of the pipe (e.g., the tubing length). If the differential pressure across a fixed section of the pressure line is monitored with a differential pressure sensor (e.g., pressure sensor 310 may be a differential pressure sensor including a P1 sensor and P2 sensor), and the inner diameter of the pressure line is known (e.g., fixed by design and stored in a non-volatile memory of the controller) and the length of tubing between P1 and P2 of the differential pressure sensor is known (e.g., fixed by design and stored in non-volatile memory of the controller), and the dynamic viscosity of the specific liquid anesthetic agent may be known and stored in a non-volatile memory of controller 108, then the flow rate of liquid anesthetic agent to the injector valve 106 can be calculated.

Turning to FIG. 5, an example of heater assembly 104 is shown. Heater assembly 104 may include heating element 316 and heating passage 314, as described above.

A first end 502 of heating element 316 may be configured to receive gas (e.g., mixed gas 118) and a second end 504 of heating element 316 may be configured to output gas (e.g., fresh gas 120). Heating passage 314 may be positioned within heating element 316 such that the received gas flows through heating passage 314 prior to exiting heating element 316 at second end 504. That is, received gas may be forced through heating passage 314, along with liquid anesthetic droplets injected into heater assembly 104 (e.g., by the injector 106). For example, an inlet 506 and an outlet 508 of heating element 316 may be positioned proximate to first end 502 and second end 504, respectively.

Heating passage 314 may be configured to transfer thermal energy from heating element 316 to the droplets of liquid anesthetic agent. As such, heating passage 314 may be constructed of a thermally conductive material, such as a metal. Heating passage 314 may be further configured to mix the received gas with the liquid anesthetic droplets. For example, geometry of heating passage 314 may form a non-linear pathway therethrough such that as the liquid anesthetic droplets are vaporized, the anesthetic vapor mixes with the received gas. Such a geometry may also increase a surface area of a heat transfer interface where the droplets contact heating passage 314, increasing spatial and energy efficiency of heating passage 314. For example, heating passage 314 may include a static mixer. Thus, heater assembly 104 may be configured for both heating and mixing (e.g., actively heating and passively mixing).

Heating passage 314 is shown with a cutout for visibility of an interior 510 thereof, which includes an example of a heating passage geometry which passively instigates gas flow patterns that result in increased mixing and expanded heat transfer interface area. An outer wall 512 may surround interior 510. For example, outer wall 512 may be a hollow cylinder shape, with open ends being inlet 506 and outlet 508. Within interior 510, there may be a plurality of guiding walls 514. Guiding walls 514 may be shaped and positioned so as to guide gaseous and liquid materials in a pathway that encourages mixing thereof. For example, guiding walls 514 may be curved to define a spiral-like pathway. Guiding walls 514 may further increase heat transfer from heating element 316 to liquid anesthetic droplets in interior 510 by increasing a surface area of conductive material to which the liquid anesthetic droplets are exposed. Thus, due to traveling through heating passage 314, the liquid anesthetic droplets may be vaporized and mixed thoroughly with the received gas.

In this way, the gas output from heater assembly 104 may include vaporized anesthetic, and may not include liquid anesthesia. Further, the outputted gas may include a homogeneous mixture of the vaporized anesthetic and the received gaseous mixture. Additionally or alternatively, heating element 316 may include an induction heater. An induction heater may provide near instantaneous heating. Heating element 316 may include the induction heater in configurations where there is a large (e.g., above a delta threshold) step change in volume of injected liquid anesthetic and/or if the inlet gas is cold (e.g., below a threshold gas temperature). All of the received gas may be included in the output gas. By heating the liquid anesthetic and gas via heater assembly 104, the liquid anesthetic may be more efficiently heated and mixed than by utilizing other heating systems which do not include a similar heating passage.

Turning to FIG. 6, a flowchart of a method 600 for control of a pressure of a cartridge pressurized anesthesia system of the present disclosure, such as cartridge pressurized anesthesia system 100 of FIGS. 1-4, 8, and 9, is shown. Instructions stored in non-volatile memory of a controller (e.g., controller 108 of FIG. 1) may be executable to perform the method 600 by sending command signals to actuators (e.g., actuators 112 of FIG. 1) responsive to feedback received from sensors (e.g., sensors 110 of FIG. 1).

At 602, the method 600 includes providing pressurized liquid anesthetic to an injector (e.g., injector 106 of FIGS. 1, 3, and 4) via a cartridge pump assembly (e.g., cartridge pump assembly 102 of FIGS. 1, 3, and 4) of the cartridge pressurized anesthesia system. The controller may send command signals to the pump (e.g., pump 304 or 404 of FIGS. 3 and 4, respectively) of the cartridge pressurized anesthesia system in order to pressurize liquid anesthesia in the cartridge (e.g., cartridge 302 or 402 of FIGS. 3 and 4, respectively), forcing the liquid anesthesia down a pressure line (e.g., pressure line 306 of FIGS. 3 and 4) between the pump and the injector. In some examples, the liquid anesthesia accumulates in a reservoir (e.g., reservoir 312 of FIGS. 3 and 4) fluidly coupled to the pressure line and upstream of the injector.

At 604, the method 600 includes measuring a pressure of the pressure line between the cartridge pump assembly and the injector. Measuring may include the controller receiving electrical signals from a gauge pressure sensor or differential pressure sensor (e.g., pressure sensor 310 of FIGS. 3 and 4) fluidly coupled to the pressure line, the signals being indicative of the pressure in the pressure line and/or drop in pressure within the pressure line across a fixed restriction (e.g., internal tubing resistance, orifice, and/or the injector valve itself). The controller may react to the signals automatically as described below.

At 606, the method 600 includes determining whether the measured pressure is equal to a target pressure. The method may determine the measured pressure is equal if the measured pressure is within a range of the target pressure. The range may be +/-5%. In an example, the range may be within +/- 5% of a nominal pressure demanded to reliably operate the injector (e.g., injector 106). A liquid injector, such injector 106, may have a characteristic minimum, nominal, and maximum inlet pressure specification. The characteristic minimum pressure may be a minimum pressure demanded for proper functioning of the liquid injector. The nominal pressure, minimum pressure, and maximum pressure may each be dependent on the ambient temperature of the anesthetic liquid. The target pressure may be specific to one or more of the type of anesthetic agent being pumped through the cartridge pressurized anesthesia system, ambient temperature of the anesthetic agent, and characteristics of the injector. For example, the controller may detect an identifier (e.g., RFID) of the cartridge and set the target pressure accordingly. Additionally or alternatively, the controller may set the target pressure based on input of a temperature sensor measuring an ambient temperature of the anesthetic agent and/or a minimum, nominal and maximum pressure of the injector stored in a non-volatile memory of the controller. In this way, the cartridge pressurized anesthesia system may be equipped to deliver various anesthetic agents having different properties.

If the measured pressure is equal to the target pressure (YES at 606), the method 600 proceeds to 608. At 608, the method 600 includes continuing to provide pressurized liquid anesthetic to an injector (e.g., without adjustment of the pressure).

Alternatively, if the measured pressure is not equal to the target pressure (NO at 606), the method 600 proceeds to 610. At 610, the method 600 includes adjusting operation of the pump of the cartridge pump assembly until the measured pressure is equal to the target pressure. For example, if the measured pressure is below the target pressure, the controller may send a command signal to the pump to increase force applied to the liquid in the cartridge (e.g., by decreasing volume of the cartridge at a faster rate). Conversely, if the measured pressure is above the target pressure, the controller may send a command signal to the pump to decrease force applied to the liquid in the cartridge (e.g., by pausing or slowing decrease in volume of the cartridge).

The method 600 returns after 608 or 610. By completing the method 600 while operating the cartridge pressurized anesthesia system of the present disclosure, a feedback loop maintains the target pressure in the pressure line. Control of feedback loops described herein (e.g., regarding methods 600 and 700 of FIGS. 6 and 7, respectively) may utilize proportional, derivative, and/or integral operations for optimized stability (e.g., minimized overshoot) and reduced delay.

Turning to FIG. 7, a flowchart of a method for control of an injector of the cartridge pressurized anesthesia system of the present disclosure, such as cartridge pressurized anesthesia system 100 of FIGS. 1-4, 8, and 9, is shown. Instructions stored in non-volatile memory of a controller (e.g., controller 108 of FIG. 1) may be executable to perform the method 700 by sending command signals to actuators (e.g., actuators 112 of FIG. 1) responsive to feedback received from sensors (e.g., sensors 110 of FIG. 1).

At 702, the method 700 includes delivering pressurized liquid anesthetic to a heater assembly (e.g., heater assembly 104 of FIGS. 1, 3, and 4) via an injector (e.g., injector 106 of FIGS. 1, 3, and 4). The injector may operate at a duty cycle responsive to command signals generated by the controller. That is, the injector may inject a fixed amount of the pressurized liquid anesthetic agent (e.g., dropwise) into the heater assembly on a frequency equal to the duty cycle.

At 704, the method 700 includes measuring a concentration of anesthetic agent in a fresh gas (e.g., fresh gas 120 of FIGS. 1, 3 and 4) output from the heater assembly. For example, the controller may receive electrical signals from an NDIR sensor (e.g., anesthetic agent sensor 322 of FIGS. 3 and 4) configured to detect the concentration. The fresh gas may flow through an inspiratory line of a ventilator as described further below. The controller may react automatically to the electrical signals originated by the sensor as described below.

At 706, the method 700 includes determining whether the measured concentration is equal to a target concentration. The method may determine the measured concentration is equal if the measured concentration is within a range of the target concentration. The range may be +/-5%. As a further example, the accuracy (e.g., the range) may be +/- 0.15 vol.% + 5% of the measured concentration. The target concentration may depend on the type of anesthetic agent being pumped through the cartridge pressurized anesthesia system. For example, a target concentration of sevoflurane may be in a range of greater than 0 vol.% to 8 vol.%, a target concentration of desflurane may be in a range of greater than 0 vol.% to 18 vol.%, and a target concentration of isoflurane may be in range of greater than 0 vol.% to 5 vol.%. Further, a response time of measuring the target concentration may be less than 160 msec. The target concentration may further depend on characteristics of the patient inhaling the fresh gas, such as age, mass, etc. As such, the target concentration may be set by a user. For example, the user may determine the target concentration and input the desired target concentration into the controller via a user interface such as user interface 124 of FIG. 1.

If the measured concentration is equal to the target concentration (YES at 706), the method proceeds to 708. At 708, the method includes continuing to deliver the pressurized liquid anesthetic agent (e.g., without adjustment of the injector duty cycle).

Alternatively, if the measured concentration is not equal to the target concentration (NO at 706), the method 700 proceeds to 710. At 710, the method 700 includes adjusting operation of the injector until the measured concentration is equal to the target concentration. For example, if the measured concentration is below the target concentration, the controller may send a command signal to the injector to increase a flow rate of liquid anesthetic agent into the heater assembly (e.g., by increasing a frequency and/or duration of the duty cycle of the injector). Conversely, if the measured concentration is above the target concentration, the controller may send a command signal to the injector to decrease the flow rate of liquid anesthetic agent into the heater assembly (e.g., by decreasing the frequency and/or duration of the duty cycle of the injector).

The method 700 returns after 708 or 710. By completing the method 700 while operating the cartridge pressurized anesthesia system of the present disclosure, a feedback loop maintains the target concentration of the vaporized anesthetic agent in the fresh gas provided to the patient.

Turning to FIG. 8, an example of a system 800 comprising cartridge pressurized anesthesia system 100 coupled to a ventilator 802 is illustrated. Ventilator 802 may be a non-limiting example of a conventional ventilator generally used in an operating room, ICU, or other medical environment. Cartridge pressurized anesthesia system 100 may adapt a ventilator system such as ventilator 802 to deliver vaporized anesthetic agents to a respiratory system of a patient 804. The system 800 may deliver various anesthetic agents to patient 804 without manual adjustment according to characteristics of the anesthetic agent. The system 800 may further eliminate exposure of the user to the anesthetic agent during refilling of the anesthetic agent. Moreover, the system 800 may eliminate disruption of anesthesia delivery during refilling.

Ventilator 802 may include an inspiratory line 808 and an expiratory line 810. Gases inhaled and exhaled by patient 804 may flow to and from patient 804 via inspiratory line 808 and expiratory line 810, respectively. Inspiratory line 808 may be coupled to a humidifier 812 adapted to provide an appropriate level of water vapor in gases inhaled by patient 804. Humidifier 812 may be fluidly coupled to an inspiratory port 814 of ventilator 802 wherefrom inhaled gases are delivered to inspiratory line 808. An expiratory port 816 of ventilator 802 may receive exhaled gases from expiratory line 810. Inspiratory line 808 and expiratory line 810 may be fluidly coupled to one or more patient lines 840 which interface directly with patient 804. Patient lines 840 may include a nasogastric tube, an endotracheal tube, and/or the like.

Cartridge pressurized anesthesia system 100 may be fluidly coupled to expiratory port 816 via a mixed gas line 818. Mixed gas may flow through mixed gas line 818 into cartridge pressurized anesthesia system 100. The mixed gas may enter cartridge pressurized anesthesia system 100 via an inlet, such as inlet 206 of FIG. 2.

As the mixed gas exhaled by patient 804 contains CO₂ which is to be removed prior to inhalation, cartridge pressurized anesthesia system 100 may include the CO₂ removal system 114. CO₂ removal system 114 may include a membrane separator configured to passively divide gaseous mixtures into two or more output streams via a membrane, where one or more of the output streams comprises CO₂ and at least one of the output streams does not include CO₂. Though described herein as a membrane separator, in other examples, an absorber or other non-membrane type separator may be used. CO₂ removal system 114 may receive mixed gas via mixed gas line 818, as well as flush gas 822. Flush gas 822 may include O₂. Flush gas 822 may further include one or more other gases in some examples, such as N₂. Flush gas 822 may flush out the CO₂ that has diffused across the membrane. Flush gas 822 may mix with CO₂ preset in mixed gas of mixed gas line 818 and exit cartridge pressurized anesthesia system 100 as waste gas 824. As such, waste gas 824 may include CO₂ in addition to any gases present in flush gas 822 such as O₂ and/or N₂. Waste gas 824 may be released into the environment or sent to scavenging system 116. Waste gas 824 may not include anesthesia gas.

The remaining mixed gas not flushed out by flush gas 822 may be provided to a vaporizing system 826. Vaporizing system 826 may include components of cartridge pressurized anesthesia system 100, such as a cartridge and pump (e.g., configured internally such as cartridge 302 and pump 304 of FIG. 3, or externally such as cartridge 402 and pump 404 of FIG. 4) adapted to provide pressurized liquid anesthetic to an injector (e.g., injector 106 of FIGS. 1, 3, and 4) which injects droplets of the liquid anesthetic into a heater assembly (e.g., heater assembly 104 of FIGS. 1, 3, and 4) to vaporize the anesthetic agent. The mixed gas flowing through mixed gas line 818 may be mixed gas 128 of FIG. 1, and the filtered (e.g., membrane separated) mixed gas provided to vaporizing system 826 which does not include CO₂ may be mixed gas 118 of FIG. 1.

A pressure limiting valve 836 may be configured to limit pressure within expiratory line 810 to protect lungs of patient 804. Pressure limiting valve 836 may be an example of pressure limiting valve 210 of FIG. 2. Pressure limiting valve 836 may be fluidly coupled to expiratory line 810 and mixed gas line 818. Pressure limiting valve 836 may selectively couple expiratory line 810 to a scavenging line 838 that leads to scavenging system 116. For example, when pressure in expiratory line 810 exceeds a threshold pressure, gas may be released into scavenging line 838 by adjusting pressure limiting valve 836 to reduce the pressure to below the threshold pressure. Consequently, a pressure of mixed gas line 818 may also be reduced. Gas may not flow through scavenging line 838 when pressure in expiratory line 810 is below the threshold. Pressure limiting valve 836 may be part of ventilator 802 and/or cartridge pressurized anesthesia system 100. In examples where ventilator 802 and cartridge pressurized anesthesia system 100 both include a pressure limiting valve (e.g., an adjustable pressure limiting valve) within the patient exhaled gas stream, the two pressure limiting valves may act as backups for each other in a dual fault tolerant configuration. Scavenging system 116 may include a container enclosing charcoal and/or one or more other materials configured to facilitate adsorption or absorption of vaporized anesthetic agent present in the gas exhaled by patient 804. The container may be periodically replaced when full, as indicated by an increase in weight of the container, as an example, allowing appropriate disposal of the materials collected therein.

Fresh gas (e.g., fresh gas 120) may be provided from cartridge pressurized anesthesia system 100 via a fresh gas line 820. Fresh gas line 820 may be fluidly coupled to cartridge pressurized anesthesia system 100 via an outlet, such as outlet 208 of FIG. 2. The fresh gas may include vaporized anesthesia agent. The fresh gas may not include CO₂. The fresh gas may not include liquid anesthesia agent. Fresh gas line 820 may be fluidly coupled to inspiratory line 808 such that patient 804 inhales gases of inspiratory line 808 and fresh gas line 820.

Gas that reaches scavenging system 116 may not be recirculated to patient 804. Instead, scavenging system 116 may remove volatile anesthetic agent from the gas, and output waste gas 122. For example, scavenging system 116 may include a container enclosing charcoal and/or other material capable of facilitating absorption or adsorption of the volatile anesthetic agent. The container may be replaced periodically, for example when an amount of increase in weight of the container indicates a maximum amount of anesthetic agent has accumulated therein. In this way, any anesthesia not metabolized by patient 804 or recirculated back to patient 804 may be disposed of without releasing anesthetic vapors into the atmosphere. In some examples, waste gas 824 output by CO₂ removal system 114 may also be routed to scavenging system 116, collecting any trace amounts of volatile anesthetic present not captured by vaporizing system 826.

Ventilator 802 may further include a controller 832. Controller 832 may be communicatively coupled to the controller of cartridge pressurized anesthesia system 100 (e.g., controller 108 of FIG. 1). As such, controller 832 and the controller of cartridge pressurized anesthesia system 100 may operate cooperatively to control system 800. The communication between controllers may occur wirelessly or via one or more electrical wires. The cartridge pressurized anesthesia system 100 may further receive electrical power from ventilator 802, in some examples via one or more electrical wires.

Ventilator 802 may further include a display 834. Display 834 may show a user parameters monitored by sensors of ventilator 802. Because controller 832 is in communication with the controller of cartridge pressurized anesthesia system 100, display 834 may further display parameters monitored by sensors of cartridge pressurized anesthesia system 100, such as concentration of anesthetic agent flowing through fresh gas line 820. That is, display 834 may be included in display 126 of cartridge pressurized anesthesia system 100 shown in FIG. 1.

In this way, cartridge pressurized anesthesia system 100 may be configured to intake gas exhaled by patient 804 and provide gas prepared for inhalation such that cartridge pressurized anesthesia system 100 is incorporated in system 800 in a closed loop configuration. By doing so, gas may be circulated through ventilator 802 and cartridge pressurized anesthesia system 100 concurrently. Further, coupling cartridge pressurized anesthesia system 100 to ventilator 802 in this way may adapt ventilator 802 to deliver various anesthetic agents with varying intrinsic characteristics. Further still, due to the configuration of cartridge pressurized anesthesia system 100 (e.g., including reservoir 312 of FIGS. 3 and 4), patient 804 may continue receiving anesthesia even while a cartridge of cartridge pressurized anesthesia system 100 is being replaced, eliminating disruptions to sedation of patient 804.

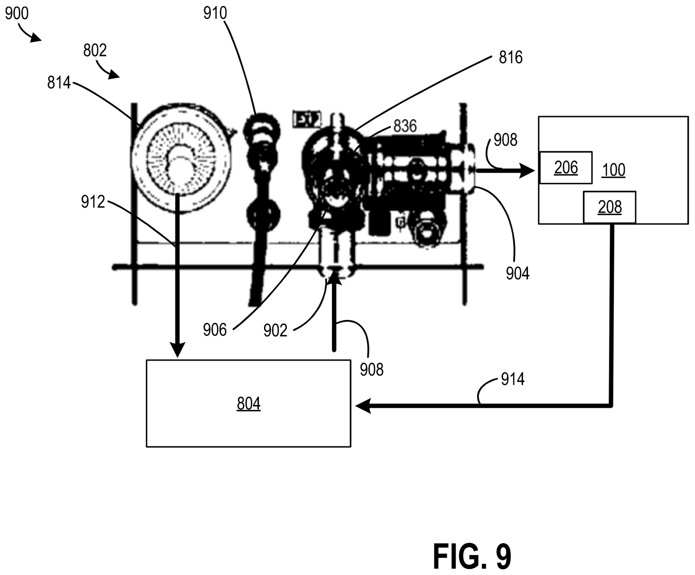

Turning to FIG. 9, a detailed view 900 of an interface of ventilator 802 and cartridge pressurized anesthesia system 100 is shown. Directions of circulatory airflow between ventilator 802, cartridge pressurized anesthesia system 100, and patient 804 are also shown.

Inspiratory port 814 may be fluidly coupled to an inspiratory line of ventilator 802 as described above. Gas (e.g., oxygenated gas) may flow from inspiratory port 814 to patient 804, as indicated by arrow 912. Gas output from ventilator 802 via inspiratory port 814 as indicated by arrow 912 and fresh gas output from cartridge pressurized anesthesia system 100 via outlet 208 as indicated by arrow 914 may combine (e.g., mix) into a gas mixture inhaled by patient 804.

Expiratory port 816 may include an inlet 902 and an outlet 904. Inlet 902 may be fluidly coupled to an expiratory line of the ventilator 802 as described above. Outlet 904 may be fluidly coupled to a mixed gas line of ventilator 802 leading to cartridge pressurized anesthesia system 100. Thus, gas may flow through expiratory port 816 in directions indicated by arrows 908. Expiratory port 816 may direct up to all gas exhaled by patient 804 to cartridge pressurized anesthesia system 100. In the case of excess pressure in the expiratory line, some gas may be directed to a scavenging system rather than being recycled into cartridge pressurized anesthesia system 100. For example, the pressure limiting valve 836 may be integral with expiratory port 816 such that pressure limiting valve 836 selectively allows gas flow through a second outlet 906 of expiratory port 816. Second outlet 906 may be fluidly coupled with the scavenging system 116 of FIGS. 1 and 8, for example via the scavenging line 838 of FIG. 8. Gas may flow out of expiratory port 816 through second outlet 906 when a pressure of the expiratory line exceeds a threshold pressure. In this way, cartridge pressurized anesthesia system 100 may utilize a pressure limiting valve of ventilator 802 to restrict pressure in the expiratory line. Alternatively, the pressure limiting valve 836 may not be integral with ventilator 802. For example, pressure limiting valve 836 may be included in closer proximity to inlet 206 than outlet 904, or in an interior of cartridge pressurized anesthesia system 100. That is, the pressure limiting valve which is configured to relieve pressure in the expiratory line by delivering gas to the scavenging system may be positioned anywhere that is downstream of patient 804 and upstream of cartridge pressurized anesthesia system 100.

Ventilator 802 may further include one or more additional ports such as a port 910 which may be fluidly coupled to inspiratory and/or expiratory lines of ventilator 802 for additional therapeutic purposes.

In this way, a closed loop is formed between ventilator 802, patient 804, and cartridge pressurized anesthesia system 100. The closed loop configuration allows recycling of anesthesia exhaled by patient 804 (e.g., anesthesia that is delivered to patient and not metabolized), reducing an amount of waste anesthesia.

In an alternative configuration, now referencing FIG. 8, cartridge pressurized anesthesia system 100 may be fluidly coupled to the inspiratory line 808. For example, cartridge pressurized anesthesia system 100 may be downstream of humidifier 812 such that humidified gas output from humidifier 812 is input to cartridge pressurized anesthesia system 100. Cartridge pressurized anesthesia system 100 may mix the humidified gas with vaporized anesthesia agent. The gas output by cartridge pressurized anesthesia system 100 may flow directly into inspiratory line 808 to patient 804. In such examples, a humidity sensor may be positioned to read a humidity of the fresh gas output by cartridge pressurized anesthesia system 100. A humidity of the fresh gas measured by the humidity sensor may be used to adjust readings of the anesthetic agent sensor 322. For example, NDIR concentration measurements in the presence of water vapor may be erroneous if a humidity based correction factor is not used. Effects of humidity on the output anesthetic agent sensor 322 may be empirically mapped and stored in a non-volatile memory of controller 108 to be used as a correction factor to the measured anesthetic agent concentration. Additionally or alternatively, a tubing permeable to water vapor (e.g., Nafion^{™}) and/or a dryer may be positioned between a sampling pump to the anesthetic agent sensor 322 and the anesthetic agent sensor 322 to drive down a relative humidity of the sampled fresh gas before it reaches the anesthetic agent sensor 322. In this way, an accurate measurement of the anesthetic agent concentration in the fresh gas may be determined.

Because the humidified gas has an acceptable CO₂ level due to function of ventilator 802, cartridge pressurized anesthesia system 100 may not include the CO₂ removal system 114, in such an example. However, anesthetic vapor exhaled by patient 804 may not be recycled. Instead all exhaled gas may be directed to scavenging system 116 as is done in regular operation of ventilator 802 without being coupled to cartridge pressurized anesthesia system 100. Thus, though fewer parts may be demanded in this example, reducing resource demand and packing volume of cartridge pressurized anesthesia system 100 while providing the effect of vaporizing anesthetics having varying properties such as boiling point, an amount of waste anesthesia accumulated in scavenging system 116 may be increased compared to the configuration shown in FIGS. 8 and 9.

Turning to FIG. 10, a flowchart of a method 1000 for operating a system including a ventilator (e.g., ventilator 802 of FIGS. 8 and 9) and a cartridge pressurized anesthesia system of the present disclosure (e.g., cartridge pressurized anesthesia system 100 of FIGS. 1-4, 8 and 9) is shown. The method 1000 may be stored as instructions in non-volatile memory of a controller of the ventilator and/or a controller of the cartridge pressurized anesthesia system. The controllers may be communicatively coupled in order to perform the method 1000 in conjunction. Additionally, one or more steps of the method 1000 may occur passively. One or more steps of the method 1000 may be performed manually by a user of the system.

At 1002, the method 1000 includes the cartridge pressurized anesthesia system receiving mixed gas from an expiratory line (e.g., expiratory line 810 of FIG. 8) of the ventilator. The mixed gas may be exhaled by the patient. As such, the mixed gas may include carbon dioxide mixed with one or more other gases.

At 1004, the method 1000 optionally includes directing gas from the expiratory line to a scavenging system (e.g., scavenging system 116 of FIGS. 1 and 8) when a pressure of the expiratory line exceeds a threshold pressure. For example, a pressure limiting valve (e.g., pressure limiting valve 210 or 836 of FIG. 2 or 8, respectively) may selectively fluidly couple the expiratory line with a scavenging line leading to the scavenging system. The scavenging system may remove anesthetic agent before releasing waste gas. The threshold pressure may be a pressure above which lungs of the patient may strained during exhale. The method 1000 may not include 1004 if the pressure does not exceed the threshold pressure.

At 1006, the method 1000 includes filtering carbon dioxide out from the mixed gas via a removal system (e.g., CO₂ removal system 114 of FIGS. 1 and 8) of the cartridge pressurized anesthesia system. The removal system may include a membrane separator in one example. Flush gas may flow through the membrane separator to flush carbon dioxide accumulated along the membrane. The carbon dioxide may be passively filtered from the mixed gas. The remaining filtered mixed gas with reduced or no CO₂ present may continue flowing through the cartridge pressurized anesthesia system while the flush gas with increased CO₂ is released (e.g., exhausted).

At 1008, the method 1000 includes combining the filtered mixed gas with an anesthetic agent vaporized via the cartridge pressurized anesthesia system to produce fresh gas. Vaporizing the anesthetic agent and combining the filtered mixed gas therewith via the cartridge pressurized anesthesia system may include pressurizing liquid anesthesia contained in a cartridge (e.g., cartridge 304 or 404 of FIGS. 3 and 4, respectively) via a pump (e.g., pump 302 or 402 of FIGS. 3 and 4, respectively), and injecting (e.g., via injector 106 of FIGS. 1, 3, and 4) droplets of the pressurized liquid anesthesia into a heater assembly (e.g., heater assembly 104 of FIGS. 1, and 3-5) where the liquid anesthesia droplets are vaporized and mixed with the filtered mixed gas via a heating passage (e.g., heating passage 314 of FIGS. 1 and 3-5). Feedback loops may be used to control operation of the injector and the pump, as described with regard to FIGS. 6 and 7. As such, 1008 may include one or more steps of the methods 600 and/or 700 of FIGS. 6 and 7, respectively.

At 1010, the method 1000 includes flowing the fresh gas into an inspiratory line of the ventilator to deliver the vaporized anesthetic agent to a patient. The fresh gas may mix with oxygenated and humidified gas from the ventilator prior to reaching the patient.

At 1012, the method 1000 includes determining whether the cartridge of the cartridge pump assembly is empty or nearly empty. For example, it may be determined that the cartridge is empty or nearly empty if a volume of the cartridge is below a threshold volume. The volume of the cartridge may correlate to a position of the pump. For example, the position of the pump, which may actuate linearly as described above, may be any position along a linear pathway of a length. The position, which can be expressed as a percentage of the length, may be used to determine the remaining cartridge volume as a percentage of the maximum volume. As such, the pump may communicate the position of the pump to the controller. When the position reaches or surpasses a threshold position, the controller may automatically determine that the cartridge is empty or nearly empty. As another example, the pump may automatically send an alert signal to the controller when the threshold position is reached or surpassed, prompting the controller to determine that the cartridge is empty or nearly empty. Alternative methods for determining the cartridge is empty or nearly empty are also possible. It may be advantageous to replace the cartridge with a full cartridge before the current cartridge is entirely empty (e.g., when nearly empty) to prevent (e.g., avoid) depletion of anesthetic liquid supply. However, due to the reservoir (e.g., reservoir 312 of FIGS. 3 and 4) of the cartridge pressurized anesthesia system being configured to hold a volume of pressurized liquid anesthetic, delivery of the anesthesia to the patient may continue without disruption for a period of time while the cartridge is empty. Thus, to minimize waste anesthesia, waiting until the cartridge is empty (e.g., when the pump is actuated to a position at an end of the length) may be desired.

If it is determined that the cartridge is not empty or nearly empty (NO at 1012), the method 1000 includes continuing to deliver the vaporized anesthetic agent to the patient at 1014.

Alternatively, if it is determined that the cartridge is empty or nearly empty (YES at 1012), the method 1000 includes replacing the cartridge while delivering the vaporized anesthetic agent to the patient at 1016. For example, the controller may automatically generate an alert that is communicated to the user via visual and/or auditory format indicating that the cartridge is empty or nearly empty. The controller may automatically fluidly decouple the cartridge from the injector by closing a liquid flow valve positioned fluidly therebetween (e.g., liquid flow valve 308 of FIGS. 3 and 4). In response to the alert, while the liquid flow valve is closed, the user may remove the cartridge and insert a new cartridge. Due to the liquid flow valve being closed, and the new cartridge fully enclosing the anesthetic liquid therein, the user is not exposed to anesthesia during the replacement. Further, because a reservoir fluidly coupled to the injector regardless of the position of the liquid flow valve is configured to hold a volume of pressurized anesthetic liquid, delivery of vaporized anesthetic to the patient may continue even while a cartridge is not fluidly coupled to the injector. When the new cartridge is in position (e.g., mechanically coupled to the pump), the controller may automatically fluidly couple the new cartridge with the injector by opening the liquid flow valve. The method 1000 returns.

The technical effect of the systems and methods disclosed herein is to provide vaporized anesthesia to a respiratory system of a patient via a cartridge pressurized anesthesia system that is compatible with various anesthetic agents having different properties (e.g., boiling point, vapor pressure, etc.) and does not cause disruptions in anesthetic delivery when replenishing supply of liquid anesthesia by replacing an empty or nearly empty cartridge with a full cartridge. To elaborate, because a volume of pressurized anesthetic is stored in a reservoir upstream of the injector, the injector may continue to inject liquid anesthetic into the heater assembly where the liquid anesthetic is vaporized while the cartridge is fluidly decoupled from the injector via a liquid flow valve. Thus, vaporization and subsequent delivery of the vaporized anesthetic to the patient may continue while the cartridge is replaced without exposing the user to the anesthesia. Further, feedback loops may allow adjustment of operation according to the anesthetic agent being delivered in a given application. As an example, a pressure of the liquid anesthetic delivered to the injector may be controlled in a first feedback loop. A concentration of vaporized anesthetic in fresh gas output from the cartridge pressurized anesthesia system may be controlled in a second feedback loop.

The disclosure also provides support for a cartridge pressurized anesthesia system, comprising: a cartridge pump assembly including a cartridge containing a liquid anesthetic configured to mechanically couple to a pump, a pressure line fluidly coupled to the cartridge, wherein the pressure line is configured to hold pressurized liquid anesthetic from the cartridge, a heater assembly fluidly coupled to the pressure line via an injector, wherein the injector disperses liquid anesthetic from the pressure line to the heater assembly, and a liquid flow valve configured to fluidly couple and decouple the cartridge and the injector. In a first example of the system, the system further comprises: a carbon dioxide removal system configured to remove carbon dioxide from mixed gas entering heater assembly. In a second example of the system, optionally including the first example, the heater assembly comprises a ceramic positive-temperature-coefficient heating element and a heating passage. In a third example of the system, optionally including one or both of the first and second examples, the cartridge is a syringe and the pump is a syringe pump. In a fourth example of the system, optionally including one or more or each of the first through third examples, the system further comprises: a pressure limiting valve upstream of the heater assembly with respect to the flow of fresh gas from the cartridge pressurized anesthesia system. In a fifth example of the system, optionally including one or more or each of the first through fourth examples, the system further comprises: an anesthetic agent sensor positioned at an output of the heater assembly and a controller including instructions stored in non-volatile memory and executable to: measure, via the anesthetic agent sensor, a concentration of in a fresh gas output from the heater assembly, and adjust an output of the injector until the measured concentration is substantially equal to a target concentration. In a sixth example of the system, optionally including one or more or each of the first through fifth examples, the system further comprises: a pressure sensor fluidly coupled to the pressure line and a controller including instructions stored in non-volatile memory and executable to: provide pressurized liquid anesthetic to the injector via the cartridge pump assembly, measure a pressure of the pressure line via the pressure sensor, and adjust operation of the pump until the measured pressure is substantially equal to a target pressure.

The disclosure also provides support for a method for operating a cartridge pressurized anesthesia system, comprising: receiving mixed gas from an expiratory line of a ventilator into the cartridge pressurized anesthesia system, combining the mixed gas with an anesthetic agent vaporized via a heater assembly of the cartridge pressurized anesthesia system to produce fresh gas, flowing the fresh gas into an inspiratory line of the ventilator to deliver the vaporized anesthetic agent to a patient, and replacing a cartridge of the cartridge pressurized anesthesia system while delivering the vaporized anesthetic agent to the patient. In a first example of the method, the anesthetic agent is provided to the heater assembly as a pressurized liquid via a cartridge pump assembly of the cartridge pressurized anesthesia system and an injector of the cartridge pressurized anesthesia system, where the injector is selectively fluidly coupled to the cartridge. In a second example of the method, optionally including the first example, combining the mixed gas with the vaporized anesthetic agent occurs passively in the heater assembly. In a third example of the method, optionally including one or both of the first and second examples, the method further comprises: filtering out carbon dioxide from the mixed gas via a removal system of the cartridge pressurized anesthesia system before combining the mixed gas with the anesthetic agent. In a fourth example of the method, optionally including one or more or each of the first through third examples, replacing the cartridge while delivering the vaporized anesthetic agent to the patient includes closing a liquid flow valve to fluidly decouple the cartridge from an injector. In a fifth example of the method, optionally including one or more or each of the first through fourth examples, the method further comprises: directing gas from the expiratory line to a scavenging system when a pressure of the expiratory line exceeds a threshold pressure.

The disclosure also provides support for a cartridge pressurized anesthesia system, comprising: a heater assembly including a gas inlet and a gas outlet, wherein the gas inlet is configured to fluidly couple to an expiratory line of a ventilator and the gas outlet is configured to fluidly couple to an inspiratory line of the ventilator, a cartridge pump assembly including a cartridge containing a liquid anesthetic agent, wherein the cartridge is mechanically coupled to a pump, a pressure line fluidly coupling the cartridge to the heater assembly via an injector, a carbon dioxide removal system positioned between the expiratory line and the gas inlet. In a first example of the system, the carbon dioxide removal system comprises a membrane separator. In a second example of the system, optionally including the first example, the system further comprises: a reservoir fluidly between the cartridge and the injector. In a third example of the system, optionally including one or both of the first and second examples, the system further comprises: a liquid flow valve closeable to fluidly separate the cartridge from the injector. In a fourth example of the system, optionally including one or more or each of the first through third examples, the cartridge pump assembly is positioned internally within a housing enclosing the injector and the heater assembly. In a fifth example of the system, optionally including one or more or each of the first through fourth examples, the system further comprises: a nondispersive infrared sensor fluidly coupled to the gas outlet. In a sixth example of the system, optionally including one or more or each of the first through fifth examples, the system further comprises: a controller communicatively coupled to the injector, the pump, the heater assembly, a pressure sensor, and an anesthetic agent sensor.

FIGS. 1-5 and 8-9 show example configurations with relative positioning of the various components. If liquid and/or gas flow is shown directly flowing from one component to another, then such elements may be referred to as directly flowing between the components, at least in one example. If shown directly contacting each other, or directly coupled, then such elements may be referred to as directly contacting or directly coupled, respectively, at least in one example. Similarly, elements shown contiguous or adjacent to one another may be contiguous or adjacent to each other, respectively, at least in one example. As an example, components laying in face-sharing contact with each other may be referred to as in face-sharing contact. As another example, elements positioned apart from each other with only a space there-between and no other components may be referred to as such, in at least one example. As yet another example, elements shown above/below one another, at opposite sides to one another, or to the left/right of one another may be referred to as such, relative to one another. Further, as shown in the figures, a topmost element or point of element may be referred to as a "top" of the component and a bottommost element or point of the element may be referred to as a "bottom" of the component, in at least one example. As used herein, top/bottom, upper/lower, above/below, may be relative to a vertical axis of the figures and used to describe positioning of elements of the figures relative to one another. As such, elements shown above other elements are positioned vertically above the other elements, in one example. As yet another example, shapes of the elements depicted within the figures may be referred to as having those shapes (e.g., such as being circular, straight, planar, curved, rounded, chamfered, angled, or the like). Further, elements shown intersecting one another may be referred to as intersecting elements or intersecting one another, in at least one example. Further still, an element shown within another element or shown outside of another element may be referred as such, in one example.

As used herein, an element or step recited in the singular and preceded with the word "a" or "an" should be understood as not excluding plural of said elements or steps, unless such exclusion is explicitly stated. Furthermore, references to "one embodiment" of the present invention are not intended to be interpreted as excluding the existence of additional embodiments that also incorporate the recited features. Moreover, unless explicitly stated to the contrary, embodiments "comprising," "including," or "having" an element or a plurality of elements having a particular property may include additional such elements not having that property. The terms "including" and "in which" are used as the plain-language equivalents of the respective terms "comprising" and "wherein." Moreover, the terms "first," "second," and "third," etc. are used merely as labels, and are not intended to impose numerical requirements or a particular positional order on their objects.

This written description uses examples to disclose the invention, including the best mode, and also to enable a person of ordinary skill in the relevant art to practice the invention, including making and using any devices or systems and performing any incorporated methods. The patentable scope of the invention is defined by the claims, and may include other examples that occur to those of ordinary skill in the art. Such other examples are intended to be within the scope of the claims if they have structural elements that do not differ from the literal language of the claims, or if they include equivalent structural elements with insubstantial differences from the literal languages of the claims.

## Claims

1. A cartridge pressurized anesthesia system (100), comprising:
a cartridge pump assembly (102) including a cartridge (302) containing a liquid anesthetic configured to mechanically couple to a pump (304);
a pressure line (306) fluidly coupled to the cartridge (302), wherein the pressure line is configured to hold pressurized liquid anesthetic from the cartridge (302);
a heater assembly (104) fluidly coupled to the pressure line via an injector (106), wherein the injector (106) disperses liquid anesthetic from the pressure line (306) to the heater assembly (104); and
a liquid flow valve (308) configured to fluidly couple and decouple the cartridge (302) and the injector (106).

2. The cartridge pressurized anesthesia system of claim 1, further comprising a carbon dioxide removal system configured to remove carbon dioxide from mixed gas entering heater assembly.

3. The cartridge pressurized anesthesia system of claim 1, wherein the heater assembly comprises a ceramic positive-temperature-coefficient heating element and a heating passage.

4. The cartridge pressurized anesthesia system of claim 1, wherein the cartridge is a syringe and the pump is a syringe pump.

5. The cartridge pressurized anesthesia system of claim 1, further comprising a pressure limiting valve upstream of the heater assembly with respect to the flow of fresh gas from the cartridge pressurized anesthesia system.

6. The cartridge pressurized anesthesia system of claim 1, further comprising an anesthetic agent sensor positioned at an output of the heater assembly and a controller including instructions stored in non-volatile memory and executable to:
measure, via the anesthetic agent sensor, a concentration of in a fresh gas output from the heater assembly; and
adjust an output of the injector until the measured concentration is substantially equal to a target concentration.

7. The cartridge pressurized anesthesia system of claim 1, further comprising a pressure sensor fluidly coupled to the pressure line and a controller including instructions stored in non-volatile memory and executable to:
provide pressurized liquid anesthetic to the injector via the cartridge pump assembly;
measure a pressure of the pressure line via the pressure sensor; and
adjust operation of the pump until the measured pressure is substantially equal to a target pressure.

8. The cartridge pressurized anesthesia system of claim 1, further comprising a reservoir fluidly between the cartridge and the injector.

9. The cartridge pressurized anesthesia system of claim 1, further comprising a liquid flow valve closeable to fluidly separate the cartridge from the injector.

10. A method for operating a cartridge pressurized anesthesia system (100), comprising:
receiving (1002) mixed gas (118) from an expiratory line (810) of a ventilator (802) into the cartridge pressurized anesthesia system (100);
combining (1008) the mixed gas (118) with an anesthetic agent vaporized via a heater assembly (104) of the cartridge pressurized anesthesia system (100) to produce fresh gas (120);
flowing (1010) the fresh gas (120) into an inspiratory line (808) of the ventilator (802) to deliver the vaporized anesthetic agent to a patient (804); and
replacing (1016) a cartridge (302) of the cartridge pressurized anesthesia system (100) while delivering the vaporized anesthetic agent to the patient (804).

11. The method of claim 8, wherein the anesthetic agent is provided to the heater assembly as a pressurized liquid via a cartridge pump assembly of the cartridge pressurized anesthesia system and an injector of the cartridge pressurized anesthesia system, where the injector is selectively fluidly coupled to the cartridge.

12. The method of claim 8, wherein combining the mixed gas with the vaporized anesthetic agent occurs passively in the heater assembly.

13. The method of claim 8, further comprising filtering out carbon dioxide from the mixed gas via a removal system of the cartridge pressurized anesthesia system before combining the mixed gas with the anesthetic agent.

14. The method of claim 8, wherein replacing the cartridge while delivering the vaporized anesthetic agent to the patient includes closing a liquid flow valve to fluidly decouple the cartridge from an injector.

15. The method of claim 8, further comprising directing gas from the expiratory line to a scavenging system when a pressure of the expiratory line exceeds a threshold pressure.
